(19) Europäisches Patentamt
European Patent Office
Office européen des brevets

(11) **EP 1 367 989 B1**

# (12) FASCICULE DE BREVET EUROPEEN

(45) Date de publication et mention de la délivrance du brevet:
**19.08.2015 Bulletin 2015/34**

(21) Numéro de dépôt: **02708434.2**

(22) Date de dépôt: **01.03.2002**

(51) Int Cl.:
**A61K 8/34** (2006.01)   **A61K 8/362** (2006.01)
**A61K 8/365** (2006.01)   **A61Q 19/00** (2006.01)

(86) Numéro de dépôt international:
**PCT/FR2002/000747**

(87) Numéro de publication internationale:
**WO 2002/072053 (19.09.2002 Gazette 2002/38)**

## (54) COMPOSITION COSMETIQUE COMPRENANT UN MELANGE ET SON UTILISATION POUR DIMINUER LA SECRETION DE SEBUM

KOSMETISCHE ZUSAMMENSETZUNG UMFASSEND EINE MISCHUNG UND VERWENDUNG ZUR VERMINDERUNG DER HAUTTALGABSONDERUNG

COSMETIC COMPOSITION COMPRISING A MIXTURE AND ITS USE TO REDUCE SECRETION OF SEBUM

(84) Etats contractants désignés:
**AT BE CH CY DE DK ES FI FR GB GR IE IT LI LU MC NL PT SE TR**

(30) Priorité: **14.03.2001 FR 0103446**

(43) Date de publication de la demande:
**10.12.2003 Bulletin 2003/50**

(73) Titulaire: **Ashland Specialties France**
**06410 Biot (FR)**

(72) Inventeurs:
• **DAL FARRA, Claude**
**F-06650 Opio (FR)**
• **DOMLOGE, Nouha**
**F-06560 Valbonne (FR)**
• **PEYRONEL, Dominique**
**F-13014 Marseille (FR)**

(74) Mandataire: **Macquet, Christophe et al**
**Macquet & Associés**
**Arche des Dolines**
**7, rue Soutrane**
**06560 Sophia Antipolis (FR)**

(56) Documents cités:
**US-A- 4 292 326**

• **M-C MARTIN ET AL.: "actifs et additifs en cosmetologie" 1993 , LAVOISIER TEC & DOC , PARIS XP002185024 196800 page 160, alinéa 3 -page 164, alinéa 1**
• **A. LEUNG ET AL.: "encyclopedia of common natural ingredients used in food, drugs and cosmetics, 2nd edition" 1996 , JOHN WILEY & SONS , NEW-YORK XP002185025 229170 page 448 -page 451**
• **DATABASE CHEMICAL ABSTRACTS [en ligne] STN; access number: 134: 76 085, XP002185026 & T. KITAHARA ET AL.: "development of omega-hydroxyl acids as sebum suppressing agents" FRAGRANCE J., vol. 28, no. 12, 2000, pages 17-21, japan**
• **DATABASE CHEMICAL ABSTRACTS [en ligne] STN; access number: 128: 39 411, XP002185027 & JP 09 315928 A (API K.K.) 9 décembre 1997 (1997-12-09)**
• **DATABASE CHEMICAL ABSTRACTS [en ligne] STN; access number: 124: 298 926, XP002185028 & JP 08 048625 A (KAO CORP.) 20 février 1996 (1996-02-20)**
• **DATABASE CHEMICAL ABSTRACTS [en ligne] STN; access number: 135: 231 481, XP002185029 & JP 2001 247433 A (POLA CHEM. IND., INC.) 11 septembre 2001 (2001-09-11)**

Remarques:
Le dossier contient des informations techniques présentées postérieurement au dépôt de la demande et ne figurant pas dans le présent fascicule.

**Description**

La présente invention se rapporte à l'utilisation dans une composition cosmétique d'une quantité efficace d'un mélange comprenant trois agents actifs : (a) l'acide sébacique, (b) l'acide 10-hydroxydécanoïque et (c) le 1,10 décanediol, à l'exclusion de la gelée royale, le mélange ou la composition étant destinés à diminuer la sécrétion de sébum.

Le sébum est produit par les sébocytes (cellules des glandes sébacées) et est ensuite sécrété à la surface de la peau. Des quantités excessives de sébum à la surface de la peau mènent à une peau dite grasse.

Dans la cosmétique moderne, de nombreuses recherches sont menées pour mettre au point des préparations afin de réduire l'aspect huileux et généralement considéré inesthétique, de la peau et des cheveux, causé par les sécrétions excessives des glandes sébacées. Il existe de nombreuses méthodes et compositions qui essayent de contrôler les sécrétions excessives de sébum, mais aucune ne s'est avérée totalement satisfaisante. Par exemple, certaines compositions utilisent des alcools gras, l'acide déhydracétique ou des salicylates anioniques. Certains shampooings, à base de souffre ou de mercure, ont été utilisés contre la séborrhée. De telles préparations présentent fréquemment, lors d'usages prolongés, des effets secondaires sans résultats vraiment satisfaisants sur la diminution du taux de sébum. En traitant la peau et les cheveux avec des préparations cosmétiques adaptées, il serait possible de réduire ces sécrétions et de rendre à la peau et aux cheveux un aspect sain.

On constate donc que subsiste le besoin d'un produit ayant une réelle action sur la diminution de la sécrétion de sébum, mais sans les inconvénients de l'art antérieur.

La demanderesse a trouvé, de façon surprenante et inattendue, qu'un mélange contenant trois agents actifs : (a) l'acide sébacique, (b) l'acide 10-hydroxydécanoïque et (c) le 1,10 décanediol, possède une activité sur la sécrétion de sébum, et que l'application d'une quantité efficace d'un tel mélange permet de diminuer la sécrétion de sébum. M-C Martin et al., "Actifs et additifs en cosmétologie", 1993, pages 160-164, Lavoisier Tec & Doc, Paris 196800 et T. Kitahara et al. "Development of omega-hydroxyl acids as sebum suppressing agents", Fragrance J., vol. 28, no. 12, 2000, pages 17-21 divulguent l'utilisation d'une composition comprenant un mélange de l'acide sébacique et l'acide 10-hydroxydécanoïque pour diminuer la sécrétion de sébum. A la connaissance de la demanderesse, il n'a jamais été décrit dans l'art antérieur l'utilisation d'un mélange contenant trois agents actifs : (a) l'acide sébacique, (b) l'acide 10-hydroxydécanoïque et (c) le 1,10 décanediol, pour diminuer la sécrétion de sébum.

La présente invention se rapporte donc à l'utilisation d'une une composition cosmétique excluant la gelée royale comprenant une quantité efficace d'un mélange comprenant trois agents actifs : (a) l'acide sébacique, (b) l'acide 10-hydroxydécanoïque et (c) le 1,10 décanediol, pour diminuer la sécrétion de sébum.

Par la suite, le terme « mélange » doit s'entendre par « mélange contenant trois agents actifs : (a) l'acide sébacique, (b) l'acide 10-hydroxydécanoïque et (c) le 1,10 décanediol ».

Par la suite, le terme « agents actifs » doit s'entendre par « (a) l'acide sébacique, (b) l'acide 10-hydroxydécanoïque et (c) le 1,10 décanediol ».

Les agents actifs utilisés représentent chacun de 10 % à 90 % du mélange tel que défini précédemment.

Le mélange, tel que défini précédemment, peut se présenter sous forme de poudre ou sous forme liquide dans un solvant adapté à la cosmétique.

Lorsque le mélange, tel que défini précédemment, se présente sous forme liquide, les agents actifs sont préalablement solubilisés dans un ou plusieurs solvants cosmétiquement ou pharmaceutiquement acceptables comme l'eau, la vaseline, une huile végétale, le propylène glycol, le butylène glycol, la glycérine, le dipropylène glycol, les diglycols éthoxylés ou propoxylés, l'éthanol, le propanol ou l'isopropanol.

Les agents actifs de la présente invention peuvent être préalablement solubilisés dans un vecteur cosmétique ou pharmaceutique comme les liposomes, ou adsorbés sur des polymères organiques poudreux, des supports minéraux comme les talcs et bentonites, et plus généralement solubilisés dans, ou fixés sur, tout vecteur cosmétiquement ou pharmaceutiquement acceptable.

Pour donner un ordre de grandeur, dans les compositions cosmétiques de la présente invention le mélange, tel que défini précédemment, est utilisé sous forme de poudre en une quantité représentant de 0,0001 % à 10 % du poids total de la composition, et préférentiellement en une quantité représentant de 0,01 % à 5 % du poids total de la composition.

Pour donner un ordre de grandeur, dans les compositions cosmétiques de la présente invention, le mélange sous forme liquide, tel que défini précédemment, contient trois agents actifs: (a) l'acide sébacique, (b) l'acide 10-hydroxydécanoïque et (c) le 1,10 décanediol, en une quantité représentant de 0,001 % à 20 % du poids total de la forme liquide, et préférentiellement en une quantité représentant de 0,1 % à 10 % du poids total de la forme liquide.

Pour donner un ordre de grandeur, dans les compositions cosmétiques de la présente invention, le mélange sous forme liquide, tel que défini précédemment, est utilisé en une quantité représentant de 0,001 % à 20 % du poids total de la composition, et préférentiellement en une quantité représentant de 0,1 % à 10 % du poids total de la composition.

Préférentiellement, les compositions selon la présente invention se présenteront sous une forme galénique adaptée à l'administration par voie topique cutanée, et couvrent toutes les formes cosmétiques. Ces compositions doivent donc contenir un milieu cosmétiquement et/ou dermatologiquement acceptable, c'est-à-dire compatible avec la peau, les poils ou les cheveux. Ces compositions pourront notamment se présenter sous forme de crèmes, émulsions huile-dans-eau, eau-dans-huile ou émulsions multiples, solutions, suspensions, ou encore poudres, adaptés à une application sur la peau, les lèvres et/ou les cheveux.

Cette composition peut être plus ou moins fluide et avoir l'aspect d'une crème, d'une lotion, d'un lait, d'un sérum, d'une pommade, d'un gel, d'une pâte ou d'une mousse. Elle peut aussi se présenter sous forme solide, comme un stick, ou être appliquée sur la peau sous forme d'aérosol. Elle peut être utilisée comme produit de soin et/ou comme produit de maquillage de la peau.

Ces compositions comprennent, de façon connue, les adjuvants nécessaires à leur formulation, tels que solvants, épaississants, diluants, anti-oxydants, colorants, filtres, pigments, charges, conservateurs, parfums, absorbeurs d'odeur. Dans tous les cas, ces adjuvants, ainsi que leurs proportions, seront choisis de manière à ne pas nuire aux propriétés recherchées dans l'invention. Ces adjuvants peuvent par exemple correspondre à 0,01 à 20 % du poids total de la composition.

Lorsque la composition de l'invention est une émulsion, la phase grasse peut représenter de 5 à 80 % en poids, et de préférence de 5 à 50 % en poids par rapport au poids total de la composition. Les émulsionnants et coémulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition.

Bien entendu, l'homme de métier veillera à choisir les éventuels composés complémentaires, actifs ou non-actifs, et/ou leurs quantités, de telle sorte que les propriétés avantageuses du mélange ne soient pas, ou sensiblement pas, altérées par l'adjonction envisagée.

Aussi, un autre objet de l'invention consiste en une composition cosmétique excluant la gelée royale, caractérisée en ce qu'elle contient, dans un milieu cosmétiquement ou dermatologiquement acceptable, un mélange comprenant trois agents actifs : (a) l'acide sébacique, (b) l'acide 10-hydroxydécanoïque et (c) le 1,10 décanediol.

Dans les compositions de l'invention, le mélange sous forme de poudre, tel que défini précédemment, est présent en une quantité représentant de 0,0001 % à 10 % du poids total de la composition, et préférentiellement en une quantité représentant de 0,01 % à 5 % du poids total de la composition.

Dans les compositions de l'invention, le mélange sous forme liquide, tel que défini précédemment, est présent en une quantité représentant de 0,001 % à 20 % du poids total de la composition, et préférentiellement en une quantité représentant de 0,1 % à 10 % du poids total de la composition.

D'autres avantages et caractéristiques de l'invention apparaîtront mieux à la lecture des exemples donnés à titre illustratif et non limitatif.

Exemple 1 - Préparation d'un mélange d'acide sébacique, d'acide 10-hydroxydécanoïque et de 1,10 décanediol

20 grammes d'acide sébacique, 20 grammes d'acide 10-hydroxydécanoïque et 20 grammes de 1,10 décanediol sont mis sous agitation pendant 4 heures dans 940 grammes de butylène glycol à 80 °C.
Le mélange est ensuite traité et stérilisé pour se conformer aux exigences cosmétiques (couleur, odeur, aspect, stérilité...). Le produit obtenu est un mélange d'acide sébacique, d'acide 10-hydroxydécanoïque et de 1,10 décanediol sous forme liquide à 6 %.

Exemple 2 - Effet du mélange de l'exemple 1 sur la diminution de la sécrétion de sébum

L'étude permet d'évaluer l'effet à court terme de l'action anti-séborrhéique du mélange de l'exemple 1.

VOLONTAIRES

10 individus de type caucasien, âgés de 29 à 50 ans (dont 5 femmes et 5 hommes), ayant une peau grasse ou mixte sur le visage, ont participé à cette étude. Elle s'est déroulée sur une journée.

ZONE D'APPLICATION DE L'ACTIF ET ZONES DE MESURE

L'étude a été réalisée en double aveugle sur le visage contre placebo. Pour chaque volontaire, le côté du visage traité avec une crème contenant 3% du mélange de l'exemple 1 a été choisi au hasard. Une étude comparative du taux de sécrétion de sébum suite à l'application de l'actif a été effectuée sur le front.
Le Sébutape donne une information précise sur le nombre de glandes sébacées (nombre de spots) ainsi que sur leur taux de sécrétion (surface totale). La pose des Sébutapes est réalisée sur la zone frontale supérieure droite et gauche.
La mesure a été pratiquée sous des conditions contrôlées de température et d'humidité. (22 °C +/- 1 et 50 % +/- 5).

PROTOCOLE EXPERIMENTAL

Tout d'abord, un nettoyage minutieux de l'épiderme a été réalisé. Un sébutape a été appliqué de chaque côté du front pendant 1 heure, afin d'indiquer le taux de sécrétion basal du sébutape.

Après cette heure, le visage du volontaire a été à nouveau délipidé et laissé au repos 15 min.

Les produits ont alors été appliqués sur les volontaires à raison de 2 mg/cm$^2$, et de nouveaux sébutapes ont été posés sur les mêmes zones que précédemment.

Au bout d'une heure, les sébutapes ont été retirés, afin d'indiquer le temps 1 heure après l'application des crèmes.

Sur les mêmes zones de mesures, des sébutapes ont été à nouveau posés pour une durée de 2 heures, puis ensuite de 3 heures correspondant au temps 3 h et 6 h après l'application des crèmes.

ANALYSE STATISTIQUE

L'analyse statistique des résultats a été pratiquée, en utilisant le test non paramétrique de Wilcoxon pour les séries appariées pour chaque sujet, sur les différences (tx-tbasal) pour les différents paramètres mesurés à l'aide des sébutapes.

où: A = Actif, P = Placebo

Les hypothèses étaient les suivantes :

Hypothèse Nulle $H_0$, aucune différence entre la moyenne enregistrée sous Placebo et celle observée sous l'Actif, c'est-à-dire

$$m_A - m_P = 0$$

Hypothèse Alternative $H_1$, la moyenne de l'actif est plus petite que celle enregistrée sous Placebo, c'est-à-dire

$$m_A < m_P$$

| | |
|---|---|
| Test statistique | Le test de Wilcoxon pour les séries appariées a été choisi car l'étude emploie deux séries appariées qui procurent des scores de différence susceptibles d'être ordonnés en fonction de leur magnitude absolue. |
| Région de rejet | Dans la mesure où le sens de la différence peut être prédit, la région de rejet sera uni-latérale. |
| Significativité | Le niveau de significativité est $\nabla \leq 5\%$. |

RESULTATS

L'analyse des sébutapes a permis de montrer l'activité du mélange de l'exemple 1 à 3% après seulement une application.

En effet, 1h après l'application des crèmes, le **nombre de spots** a diminué en présence du mélange de l'exemple 1 à 3% (T1heure - Tbasal) = -12,5, alors qu'il a augmenté sur la zone du placebo (T1heure - Tbasal) = +16,5. Les résultats obtenus sont significatifs pour le nombre de spots (p = 0.030).

La **surface totale des spots** est plus importante au temps 3h et 6h par rapport au temps 1h. En effet les temps 1h, 3h et 6h après l'application des crèmes correspondent respectivement à un temps de sécrétion sur le sébutape de 1h, 2h et 3h. Mais, quel que soit le temps d'étude, la différence est toujours en faveur de l'actif en ce qui concerne la surface totale.

(T1heure - Tbasal) est de - 3,3 mm$^2$ en présence du mélange contre + 1,2 mm$^2$ pour le placebo.

(T3heure - Tbasal) est de + 1,3 mm$^2$ en présence du mélange contre + 4,0 mm$^2$ pour le placebo.

(T6heure - Tbasal) est de + 4,1 mm$^2$ en présence du mélange contre + 5,4 mm$^2$ pour le placebo.

Cette différence est hautement significative pour le temps 1h (p = 0.001) et significative pour le temps 3h (p = 0.0463).

CONCLUSION

On note une nette diminution du nombre et de la surface totale des spots après une seule application du mélange de l'exemple 1 à 3 %, et cela dès 1 heure après l'application.

Exemple 3 - Préparation de compositions

Ces compositions ont été obtenues par simple mélange des différents composants. Les quantités indiquées sont données en pourcentage de poids.

**1-Emulsion huile dans eau**

Phase huileuse :

- Montanov 68 (Cetearyl Alcohol and Cetearyl Glucoside)     5,00 %
- Huile de Jojoba      5,00 %
- Huile de Vaseline       5,00 %
- Isopropyl Palmitate       7,00 %

Phase aqueuse :

- Glycérine       5,00 %
- Allantoïne      0,10 %
- Mélange de l'exemple 1       3,00 %
- Sepigel 305 (Polyacrylamide and C13-14 Isoparaffin and Laureth-7)       0,30 %
- Conservateur      0,50 %
- Parfum       0,50 %
- Eau qsp       100 %

**2- Gel**

- Carbopol Ultrez 10 (sol. à 2%)       25,00 %
- Triéthanolamine      0,50 %
- mélange de l'exemple 1       1,0 %
- Conservateur      0,20 %
- EDTA (séquestrant)       0,10 %
- Parfum       0,50 %
- Eau qsp       100 %

**3- Lotion**

- Mono Propylene Glycol       1,00 %
- Allantoïne      0,30 %
- Glycérine      1,00 %
- Cetiol HE (PEG-7 Glyceryl Cocoate)       1,00 %
- mélange de l'exemple 1       5,00 %
- Conservateur      0,20 %
- Parfum       0,50 %
- Eau qsp       100 %

**Revendications**

1. Utilisation d'une composition cosmétique excluant la gelée royale comprenant une quantité efficace d'un mélange comprenant trois agents actifs : (c) le 1,10 décanediol, (a) l'acide sébacique et (b) l'acide 10-hydroxydécanoïque, pour diminuer la sécrétion de sébum.

2. Utilisation selon la revendication 1, **caractérisée en ce que** le mélange se présente sous forme de poudre ou sous forme liquide dans un solvant adapté à la cosmétique.

3. Utilisation selon la revendication précédente, **caractérisée en ce que** lorsque le mélange se présente sous forme liquide, les trois agents actifs sont préalablement solubilisés dans un ou plusieurs solvants cosmétiquement ou pharmaceutiquement acceptables choisis parmi l'eau, la vaseline, une huile végétale, le propylène glycol, le butylène glycol, la glycérine, le dipropylène glycol, les diglycols éthoxylés ou propoxylés, l'éthanol, le propanol ou l'isopropanol.

4. Utilisation selon la revendication 2, **caractérisée en ce que** lorsque le mélange se présente sous forme de poudre, les trois agents actifs sont préalablement solubilisés dans un vecteur cosmétique ou pharmaceutique choisi parmi les liposomes, ou adsorbés sur des polymères organiques poudreux, ou des supports minéraux choisis parmi les talcs et bentonites.

5. Utilisation selon la revendication 4, **caractérisée en ce que** le mélange est utilisé sous forme de poudre en une quantité représentant de 0,0001 % à 10 % du poids total de la composition.

6. Utilisation selon la revendication 3, **caractérisée en ce que** le mélange sous forme liquide contient les trois agents actifs en une quantité représentant de 0,001 % à 20 % du poids total de la forme liquide.

7. Utilisation selon la revendication 6, **caractérisée en ce que** le mélange sous forme liquide contient les trois agents actifs en une quantité représentant de 0,1 % à 10 % du poids total de la forme liquide.

8. Utilisation selon la revendication 6 ou 7, **caractérisée en ce que** le mélange sous forme liquide est utilisé en une quantité représentant de 0,001 % à 20 % du poids total de la composition.

9. Composition cosmétique excluant la gelée royale, **caractérisée en ce qu'**elle contient, dans un milieu cosmétiquement ou dermatologiquement acceptable, un mélange comprenant trois agents actifs : (c) le 1,10 décanediol, (a) l'acide sébacique et (b) l'acide 10-hydroxydécanoïque.

10. Composition selon la revendication précédente, **caractérisée en ce que** le mélange se présente sous forme de poudre dans lequel les agents actifs sont préalablement solubilisés dans un vecteur cosmétique ou pharmaceutique choisi parmi les liposomes, ou adsorbés sur des polymères organiques poudreux, des supports minéraux choisis parmi les talcs et bentonites, et est présent en une quantité représentant de 0,0001 % à 10 % du poids total de la composition.

11. Composition selon la revendication 10, **caractérisée en ce que** le mélange sous forme de poudre est présent en une quantité représentant de 0,01 % à 5 % du poids total de la composition.

12. Composition selon la revendication 9, **caractérisée en ce que** le mélange se présente sous forme liquide dans lequel les agents actifs sont préalablement solubilisés dans un ou plusieurs solvants cosmétiquement ou pharmaceutiquement acceptables choisis parmi l'eau, la vaseline, une huile végétale, le propylène glycol, le butylène glycol, la glycérine, le dipropylène glycol, les diglycols éthoxylés ou propoxylés, l'éthanol, le propanol ou l'isopropanol, et est présent en une quantité représentant de 0,001 % à 20 % du poids total de la composition.

13. Composition selon la revendication 12, **caractérisée en ce que** le mélange sous forme liquide est présent en une quantité représentant de 0,1 % à 10 % du poids total de la composition.

**Patentansprüche**

1. Verwendung einer kosmetischen Zusammensetzung mit Ausnahme des Gelee Royale, umfassend eine wirksame Menge einer Mischung, umfassend drei Wirkstoffe: (c) 1,10-Decandiol, (a) Sebacinsäure und (b) 10-Hydroxydecansäure, um die Hauttalgabsonderung zu verringern.

**2.** Verwendung nach Anspruch 1, **dadurch gekennzeichnet, dass** die Mischung die Form eines Pulvers oder eine flüssige Form in einem Lösemittel aufweist, das für die Kosmetik geeignet ist.

**3.** Verwendung nach dem vorhergehenden Anspruch, **dadurch gekennzeichnet, dass** wenn die Mischung in flüssiger Form vorliegt, die drei Wirkstoffe zuvor in einem oder in mehreren kosmetisch oder pharmazeutisch akzeptablen Lösemitteln solubilisiert werden, ausgewählt aus Wasser, Vaselin, pflanzlichem Öl, Propylenglykol, Butylenglykol, Glycerin, Dipropylenglykol, ethoxylierten oder propoxylierten Diglykolen, Ethanol, Propanol oder Isopropanol.

**4.** Verwendung nach Anspruch 2, **dadurch gekennzeichnet, dass** wenn die Mischung die Form eines Pulvers aufweist, die drei Wirkstoffe zuvor in einem kosmetischen oder pharmazeutischen Vektor solubilisiert werden, ausgewählt aus Liposomen oder adsorbiert auf pulvrigen organischen Polymeren oder mineralischen Trägern, ausgewählt aus den Talks und Bentoniten.

**5.** Verwendung nach Anspruch 4, **dadurch gekennzeichnet, dass** die Mischung in Form eines Pulvers und in einer Menge verwendet wird, die 0,0001 % bis 10 % des Gesamtgewichts der Zusammensetzung darstellt.

**6.** Verwendung nach Anspruch 3, **dadurch gekennzeichnet, dass** die Mischung in flüssiger Form die drei Wirkstoffe in einer Menge enthält, die 0,001 % bis 20 % des Gesamtgewichts der flüssigen Form darstellt.

**7.** Verwendung nach Anspruch 6, **dadurch gekennzeichnet, dass** die Mischung in flüssiger Form die drei Wirkstoffe in einer Menge enthält, die 0,1 % bis 10 % des Gesamtgewichts der flüssigen Form darstellt.

**8.** Verwendung nach Anspruch 6 oder 7, **dadurch gekennzeichnet, dass** die Mischung in flüssiger Form in einer Menge verwendet wird, die 0,001 % bis 20 % des Gesamtgewichts der Zusammensetzung darstellt.

**9.** Kosmetische Zusammensetzung mit Ausnahme des Gelee Royale, **dadurch gekennzeichnet, dass** sie, in einem kosmetisch oder dermatologisch akzeptablen Medium, eine Mischung enthält, die drei Wirkstoffe umfasst: (c) 1,10-Decandiol,(a) Sebacinsäure und (b) 10-Hydroxydecansäure.

**10.** Zusammensetzung nach dem vorhergehenden Anspruch, **dadurch gekennzeichnet, dass** die Mischung die Form eines Pulvers aufweist, in dem die Wirkstoffe zuvor in einem kosmetischen oder pharmazeutischen Vektor solubilisiert werden, ausgewählt aus den Liposomen oder adsorbiert auf pulvrigen organischen Polymeren mineralischen Trägern, ausgewählt aus Talks und Betoniten, und in einer Menge vorhanden ist, die 0,0001 % bis 10% des Gesamtgewichts der Zusammensetzung darstellt.

**11.** Zusammensetzung nach Anspruch 10, **dadurch gekennzeichnet, dass** die Mischung in Form eines Pulvers in einer Menge vorhanden ist, die 0,01 % bis 5 % des Gesamtgewichts der Zusammensetzung darstellt.

**12.** Zusammensetzung nach Anspruch 9, **dadurch gekennzeichnet, dass** die Mischung eine flüssige Form aufweist, in der die Wirkstoffe zuvor in einem oder in mehreren kosmetisch oder pharmazeutisch akzeptablen Lösemitteln solubilisiert werden, ausgewählt aus Wasser, Vaselin, einem pflanzlichen Öl, Propylenglykol, Butylenglykol, Glycerin, Dipropylenglykol, ethoxylierten oder propoxylierten Diglykolen, Ethanol, Propanol oder Isopropanol, und in einer Menge vorhanden ist, die 0,001 % bis 20 % des Gesamtgewichts der Zusammensetzung darstellt.

**13.** Zusammensetzung nach Anspruch 12, **dadurch gekennzeichnet, dass** die Mischung in flüssiger Form in einer Menge vorhanden ist, die 0,1 % bis 10 % des Gesamtgewichts der Zusammensetzung darstellt.

**Claims**

**1.** Use of a cosmetic composition excluding royal jelly comprising an effective quantity of a mixture comprising three active agents: (c) 1,10 decanediol, (a) sebacic acid and (b) 10-hydroxydecanoic acid, to reduce the secretion of sebum.

**2.** Use according to claim 1, **characterised in that** the mixture is presented in powder form or in liquid form in a solvent suitable for cosmetics.

**3.** Use according to the above claim, **characterised in that**, when the mixture is presented in liquid form, the three active agents are previously solubilised in one or a plurality of cosmetically or pharmaceutically acceptable solvents chosen from water, petrolatum, a plant oil, propylene glycol, butylene glycol, glycerine, dipropylene glycol, ethoxylated or propxylated diglycols, ethanol, propanol or isopropanol.

**4.** Use according to claim 2, **characterised in that**, when the mixture is presented in powder form, the three active agents are previously solubilised in a cosmetic or pharmaceutical carrier chosen from liposomes, or adsorbed on organic polymer powders, or mineral substrates chosen from talcs and bentonites.

**5.** Use according to claim 4, **characterised in that** the mixture is used in powder form in a quantity representing from 0.0001% to 10% of the total weight of the composition.

**6.** Use according to claim 3, **characterised in that** the mixture in liquid form contains the three active agents in a quantity representing from 0.001% to 20% of the total weight of the liquid form.

**7.** Use according to claim 6, **characterised in that** the mixture in liquid form contains the three active agents in a quantity representing from 0.1% to 10% of the total weight of the liquid form.

**8.** Use according to claim 6 or 7, **characterised in that** the mixture in liquid form is used in a quantity representing from 0.001% to 20% of the total weight of the composition.

**9.** Cosmetic composition excluding royal jelly, **characterised in that** it contains, in a cosmetically or dermatologically acceptable medium, a mixture comprising three active agents: (c) 1,10 decanediol, (a) sebacic acid and (b) 10-hydroxydecanoic acid.

**10.** Composition according to the above claim, **characterised in that** the mixture is presented in powder form wherein the active agents are previously solubilised in a cosmetic or pharmaceutical carrier chosen from liposomes, or adsorbed on organic polymer powders, or mineral substrates chosen from talcs and bentonites, and is present in a quantity representing from 0.0001% to 10% of the total weight of the composition.

**11.** Composition according to claim 10, **characterised in that** the mixture in powder form is present in a quantity representing from 0.01% to 5% of the total weight of the composition.

**12.** Composition according to claim 9, **characterised in that** the mixture is presented in liquid form wherein the active agents are previously solubilised in one or a plurality of cosmetically or pharmaceutically acceptable solvents chosen from water, petrolatum, a plant oil, propylene glycol, butylene glycol, glycerine, dipropylene glycol, ethoxylated or propxylated diglycols, ethanol, propanol or isopropanol, and is present in a quantity representing from 0.001% to 20% of the total weight of the composition.

**13.** Composition according to claim 12, **characterised in that** the mixture in liquid form is present in a quantity representing from 0.1% to 10% of the total weight of the composition.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- Actifs et additifs en cosmétologie. **M-C MARTIN et al.** Actifs et additifs en cosmétologie. Lavoisier Tec & Doc, 1993, 160-164

- **T. KITAHARA et al.** Development of omega-hydroxyl acids as sebum suppressing agents. *Fragrance J.,* 2000, vol. 28 (12), 17-21